# EUROPEAN PATENT SPECIFICATION

(11) **EP 0 428 331 B1**
(45) Date of publication and mention of the grant of the patent: **25.05.1994**
(21) Application number: 90312214.1
(22) Date of filing: 08.11.1990
(51) Int. Cl.: F16K 5/04, F16K 5/02, A61F 5/44

(54) **Fluid control valve**
Flüssigkeitsregelventil
Vanne de réglage pour fluide

(30) Priority: 09.11.1989 GB 8925319
(43) Date of publication of application: 22.05.1991
(73) Proprietor: BARD LIMITED, Crawley West Sussex RH11 9BP (GB)
(72) Inventor: Deacon, Brian Alistair, Billericay, Essex CM12 9NB (GB); Hunter, Richard John, Clacton-on-Sea, Essex C015 (GB)
(74) Representative: Jackson, Peter

(56) References cited:
- DE-A- 3 437 490
- GB-A- 613 852
- GB-A- 2 166 222

## Description

This invention relates to a fluid control valve and in particular to a valve with an alignment of fluid flow conduits which facilitates manipulation of the valve between its open and closed positions.

Developments in fluid control valves in recent years have been largely directed to improvements in operational convenience. For example in domestic water supplies to such appliances as clothes- and dishwashing machines the traditional faucet tap with a washer and seating configuration, which requires several turns of a screw handle for full opening, has been widely replaced by a simple action quarter-turn valve in which a cylindrical stopper with a conduit through the centre is rotated between an alignment which permits full fluid flow and an alignment which prevents it. Such improvements have been greatly assisted by the increasing use of plastics in valve construction.

Heavy duty taps, such as stop-cocks, are described and claimed in such documents as DE-A-3437490 and GB-A-613852. DE-A-3437490 discloses a stop-cock in which the fluid flow conduit through its rotatable stop plug is offset so as to give as large a flow passage as possible through the plug. The rotatable plug is preferably constructed of ceramic material but can alternatively be metal. GB-A-613852 discloses a shut-off valve for fluid under pressure, and also has an offset flow passage through its rotatable plug, but in this case the passage is offset so that the rotary plug can accommodate a spring-loaded sealing unit which is activated by pressure in the fluid flow passage. These heavy duty units do not incorporate an operating lever but instead have a short protruding shaft to which an operating device can be applied when required.

One specialist medical field in which there has been much development work on simplified valve configurations is that of drainage valves for body fluids. A typical application of these is the valve used in combination with a urine collection bag connected to a bladder drainage catheter. For reasons of hygiene the collection bag and valve have a relatively short service life before disposal and thus should be of simple and inexpensive construction while nevertheless providing a reliable seal against leakage.

The present invention relates to a valve which is particularly suitable for use in such medical applications and the invention is described herein primarily with reference to such uses. It is however to be understood that the valve according to the invention is also beneficial in other applications, as discussed later in this description.

A high proportion of patients requiring urine drainage catheters have limited manual dexterity such that they find it difficult to operate an associated drainage valve. The open and close action must therefore be easy to accomplish both in terms of allowing easy finger engagement and in requiring a small amount of effort to move the valve between the open and closed positions. A further requirement is that the patient or carer should be able to determine clearly whether the valve is in the open or closed position.

Our earlier patent specification GB-A-2129912 relates to a bladder drainage valve comprising a valve body and a rotatable insert in which the insert is retained in place by an external bridging portion which also serves as the operating limb to open and close the valve. The insert and bridging portion can be formed as a single moulding of a plastics material. The specification describes the provision of a relatively large finger contact surface on the valve-operating limb, and the use of complementary low friction plastics materials for the valve body and the rotatable insert.

As with other prior proposals for an easy open-close action of such a rotatable insert our prior specification describes a configuration in which the operating limb or lever moves through an angle of substantially 90° between the valve-open and valve-closed positions. With a 90° action, however, the operating lever stands proud of the valve in one or both of these positions. The preference in our earlier specification is for the lever to stand proud when the valve is in the open position and not to stand proud in the closed position. This configuration works particularly well for the outlet valve of a leg-mounted bag worn by an ambulatory patient under clothing since the valve lever is merely opened for periodic drainage and is mostly in the closed, non-proud, position in which it does not snag the clothing. The protruding lever also provides a clear indication that the valve is in the open position and thus acts as a reminder to the patient or carer to reclose it before leaving the drainage point. In other applications, however, in which it may be necessary to leave the valve in the open position for longer periods, for example in drainage systems connected to bedside collection bags and especially in systems for overnight use and for bedridden patients, the protruding lever presents increased risks of snagging the bedclothes, of inadvertent closure of the valve or of causing discomfort to the patient.

A primary object of the present invention is accordingly to provide a low profile valve having an operating lever which can be located in a non-proud alignment in both the open and closed positions of the valve. A further object is to provide a valve which can be easily operated by a person of limited manual dexterity.

According to the invention there is provided a fluid control valve which comprises a housing having a generally cylindrical or frusto-conical inner surface with a fluid inlet port and fluid outlet port therein, a fluid inlet tube and a fluid outlet tube leading respectively to and from the fluid inlet port and fluid outlet port, and a tap member having a fluid flow conduit therethrough and having an outer surface of complementary shape to the inner surface of the housing so as to be movable therein about an axis of rotation between an open position in which its fluid flow conduit is in alignment with the fluid inlet port and fluid outlet port and a closed position in which its fluid flow conduit is out of alignment with at least one of the ports, the fluid flow conduit being offset relative to the axis of rotation of the tap member, characterised in that the tap member has a lever attached thereto which is rotatable over a wide angle so as to be aligned close to the inlet tube or outlet tube in both the open and closed positions.

For convenience of description herein the alignment of each of the fluid flow conduit through the tap member and the fluid inlet tube and fluid outlet tube is described with reference to a line joining the central points of the cross-sectional area of the respective conduit or tube throughout its length. In the preferred case discussed below of circular internal cross section and straight line construction the central line is the axis of the conduit or tube. References herein to the offset disposition of the said conduit or tubes are made in relation to the position of the said central line relative to the axis of rotation of the tap member. The requirement of the invention is that the said central line must be out of alignment with the axis of rotation. In conventional cylindrical valves the central line of the fluid flow conduit intersects the axis of rotation.

The said requirement of the invention for offset disposition of the fluid flow conduit can alternatively be expressed with reference to the valve housing in that a straight line passing through the centres of both the inlet and outlet ports in the generally cylindrical or frusto-conical inner surface of the housing must be offset (i.e. out of alignment) relative to the axis of the said surface. This axis of the inner surface of the housing necessarily coincides with the axis of rotation of the tap member.

The offset disposition of the fluid flow conduit according to the invention offers great flexibility of choice in the angle through which the valve-operating lever is rotated between its valve-open and valve-closed position. In particular it permits the lever to be rotated over a wide angle such that in both its open and closed positions it is aligned close to the inlet and outlet tubes and thus presents a low profile in both positions. In addition to the low profile the wide-angle action offers the advantages of ready manipulation of the lever to open and close the valve. Because of the low-profile alignment of the lever it can be made longer than in prior versions and thereby reduce the effort to be applied to its operating tab. It further permits a clear visual indication of whether the valve is in the open or closed position.

As discussed above, the offset position of the fluid conduit in the tap member dictates that the alignment of the inlet and outlet ports relative to each other represents a path across the housing which is also offset from the axis of rotation of the tap. This alignment allows the inlet and outlet tubes to be located closer than a conventional valve to an adjacent surface and thus permits the use of this new type of valve in piping systems where the proximity of the piping to the surface would prevent the use of a conventional valve.

The operating lever can conveniently be attached to just one end of the tap member, thereby permitting the housing to be closed off at the other end of the tap member, or can be attached to both ends of the tap member, for example by a bridging portion as described in our earlier patent discussed above. In all configurations the lever is preferably fitted with a handling tab to assist its movement. The handling tab preferably includes at least one angled or curved portion at its outer end, i.e the end remote from the tap member, so as to assist the user in hooking a finger or an implement, such as a metal spatula or other instrument, under the tab. If desired the tab can also include as a further aid to movement a slot or orifice to receive such an implement.

The handling tab is preferably aligned in a common or parallel plane to the axis of rotation of the tap member. This not only assists in easy manipulation of the lever but also offers the advantage of permitting one surface to face the user when the valve is in the open position and a different surface when the valve is in the closed position. The lever can if required carry distinguishable markings on the different surfaces, for example on one surface the word "OPEN" or a first colour or pattern and on the other surface the word "CLOSED" or a different colour or pattern.

The surface of rotation of the tap and the inner curved surface of the housing within which the tap rotates should be of closely similar shape. In general it is preferred that these surfaces should both be substantially cylindrical, the cylinders being each of uniform diameter along the axis of rotation. Alternatively, if the configuration of valve or associated pipework so requires, these surfaces can be of complementary frusto-conical or stepped cylindrical shapes. In all cases these surfaces should be a close fit, desirably an interference fit, with each other. Such closely similar surface shapes are required so as to assist in providing a leak-tight fit while permitting ease of rotation. A semi-fluid sealant and a lubricant are preferably applied to at least one of the surfaces during assembly of the valve. The sealant properties are required as a further safeguard against fluid leakage. The lubricant properties help to ensure ease of rotation. Silicone greases are well suited to satisfying both of these duties, and are suitable for use with urine.

Additionally the housing and tap member preferably have complementary circumferential ridges and grooves at the open end (or ends) of the housing. These hold the housing and tap member in a fixed position along the axis of rotation.

In order to ensure that the required shapes for the housing and tap member are reliably achieved they should preferably be constructed from materials which are capable of being precision engineered. Preferably both the housing and tap member are plastics mouldings, precision engineered as required to the required shape. Metals such as stainless steel and aluminium are also well suited to the required duty but tend to be expensive compared with the plastics alternatives.

Suitable plastics materials for the housing include high density polyethylene, unplasticised polyvinyl chloride and ABS acrylonitrile-butadiene-styrene copolymers. The plastics material for the tap member is preferably different from that of the housing, and can for example be polypropylene or filled polypropylene. If desired a single plastics moulding can be used for the tap member, lever and finger tab. Similarly a single moulding can be used for the housing, inlet and outlet ports and inlet and outlet tubes.

The fluid inlet and outlet tubes and the fluid flow conduit through the tap are preferably all of a shape and alignment which permits laminar fluid flow through them. As indicated above, all three are preferably of circular internal cross section, preferably straight throughout their length and preferably of substantially the same internal cross sectional area throughout their length (although they may have a slight taper along their length to assist in manufacture). They are preferably aligned along a common axis which will usually most conveniently be at right angles to a plane passing through the axis of rotation of the tap member.

The fluid flow conduit through the tap member can conveniently be in the form of a bore but can alternatively be in the form of a groove, generally of U-shape. A bore construction is easy to fabricate, especially in a plastics material, and can be readily formed to substantially the same cross-sectional shape and area as the inlet and outlet tubes, thereby providing for laminar flow of fluid into, through and out of the valve. Laminar flow offers the advantage of maximising the rate of flow of fluid through the valve, and thus of maximising the rate of drainage.

A groove configuration of the tap member is also simple to manufacture and offers the advantage that the inlet and outlet tubes can be aligned and located close to the curved surface of the housing. This in turn means that the housing and tubes can be located very close to an adjacent surface. It is a particularly suitable configuration if the cross sectional area of the inlet and outlet tubes is large relative to the diameter of the tap member since it allows a major portion of the cross-sectional area of the tap member to be occupied by the fluid path while ensuring the maximum extent of offset disposition of the conduit.

With reference to the centre line of the conduit through the tap member (referred to herein as the "fluid flow line") and the axis of rotation of the tap member, the fluid flow line is preferably offset from the axis of rotation by a minimum of 20% of the radial distance from the axis of rotation to the circumference of the tap member. With less offset than the said 20% the operating lever tends to protrude to an unacceptable degree. The maximum extent to which the fluid flow line can be offset is determined by the cross sectional area of the fluid flow conduit: the larger the diameter of the conduit the smaller is the possible extent of offset. For example for a conduit with a diameter greater than the said radial distance of the tap member the maximum offset, expressed in the terms defined above, is less than 50%. It will also be noted that for relatively large cross sectional areas of the fluid flow conduit while the centre line is offset from the axis of rotation part of the peripheral portion of the fluid flow will pass through the axis of rotation.

As measured in a plane which passes through the tap member at right angles to the axis of rotation and coincides with the centre line of the fluid flow conduit, the cross sectional area of the fluid flow conduit should generally be in the range of 35 to 80% of the cross sectional area of the tap member. If the fluid flow conduit has a smaller cross sectional area than the said 35% the fluid flow tends to be unduly restricted and the valve housing is large relative to the maximum fluid flow through the valve and thus may be unduly obtrusive. If the fluid flow conduit has a larger cross sectional area than the said 80% there may be insufficient closed surface area on the tap to block the fluid flow path when the valve is in the closed position.

Such a large conduit may also unduly weaken the tap member and thus permit distortion of the tap member in use such as to permit fluid leakage. The preferred cross sectional area of the fluid flow conduit is in the range 50 to 80% of the cross sectional area of the tap member.

Whereas the comparable valves of the prior art had an operational angle of about 90°, those of the invention with the preferred configurations of handling tab can increase the operating angle to at least 150°. In versions of the invention without a handling tab or with a handling tab projecting away from the fluid inlet and outlet tubes, the operating lever can be moved between a position alongside the inlet tube to a position alongside the outlet tube, giving an operating angle of 180°.

The outer configuration of the tubes depends upon the intended purpose of the valves. For urine drainage a smooth surface allows easy connection to associated plastic drainage tubes or bags, possibly together with the assistance of a solvent bond or weld. For other duties a threaded surface or the presence of pins and slots may be more appropriate. For most duties the outlet tube preferably has an angled outlet so as to discourage retention of a fluid layer therein by instead assisting formation and discharge of fluid droplets.

In addition to their medical uses valves according to the invention are well suited to general plumbing duties, both for water and gas pipework. The use of tab labelling permits easy inspection of whether a valve is in the open or closed position. A particularly useful application is for emergency showers in which a single positive pulling action on the operating lever releases a spray of washing water for victims of burns or chemical spillages. The tab arrangement allows clear labelling of the emergency action required and the position at which it is to be taken.

The invention is further illustrated below with reference to the accompanying figures, in which
Figure 1 is a general view of a urine drainage valve according to the invention,
Figure 2 is a sectional view of the Figure 1 valve (as seen from Y-Y on Figure 1),
Figure 3 is a sectional view of the Figure 1 valve (on the line X-X of Figure 2), and
Figure 4 is a detailed view, on a slightly larger scale than the other figures, of an insert portion of the Figure 1 valve.

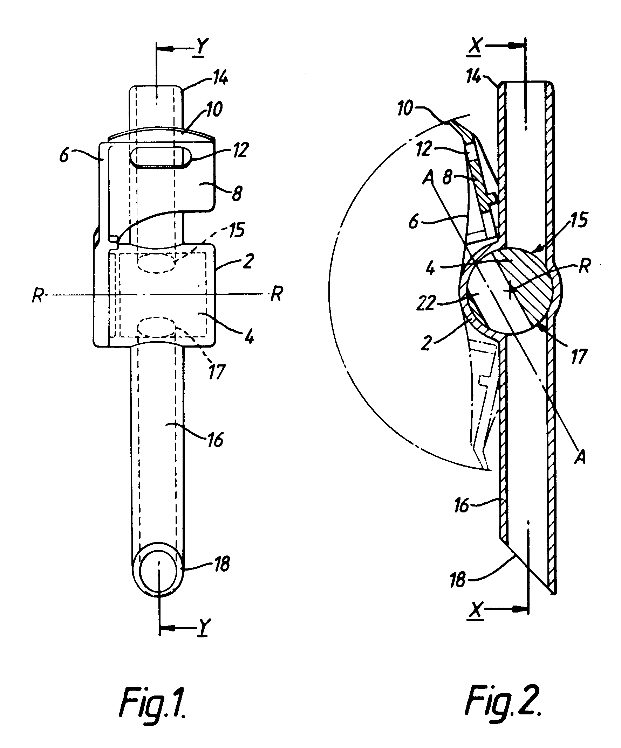

The valve includes a cylindrical housing 2 with a cylindrical tap member 4 rotatably mounted therein. As viewed in Figure 1, the right hand side of the housing 2 is closed, the left hand side is open and an operating lever 6 is attached to the left hand side of the tap member 4. The lever 6 has a lateral tab 8 with an angled outer portion 10 and a slot 12, both to assist movement of the lever 6.

An inlet tube 14 leads to the housing 2 from above and enters the housing through an inlet port 15. Similarly an outlet tube 16 leads from an outlet port 17 in the housing 2. As is most clearly seen in Figure 2, the tubes 14 and 16 are in straight line alignment with each other and are offset from the axis of rotation R-R of the tap member 4.

The lower end of the tube 16 has an an angled portion 18 to assist drops of urine to form and fall from the tube 16.

The tap member 4 has a shoulder 24 to bear against the left hand edge of the housing 2 and a circumferential ridge 26 to engage in a corresponding slot in the inner surface of the housing 2, both features being intended to keep the tap member 4 in place in the housing 2.

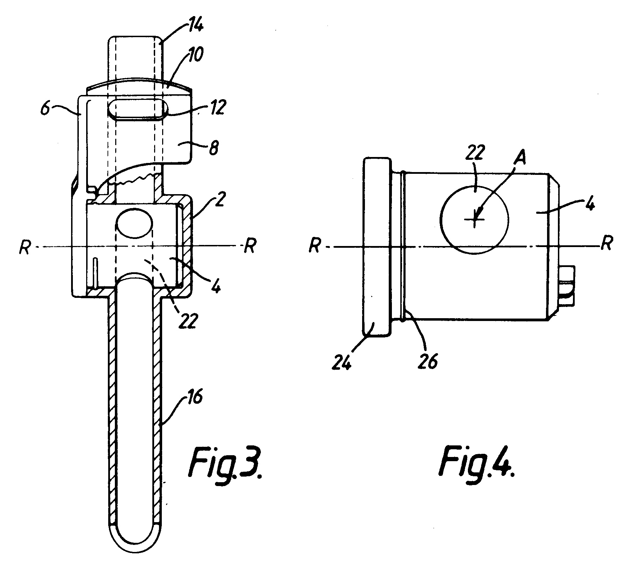

The tap member 4 includes a cylindrical bore 22. The central line (axis) A-A of the bore 22, which in the valve-open position coincides with the axis of the inlet and outlet tubes 14 and 16, is offset from the axis of rotation R-R by a distance measuring 36% of the radius of the tap member 4. Figure 2 is viewed in a direction along the axis of rotation R-R of the tap member 4 and Figure 4 is viewed in a direction along the axis A-A of the bore 22 such that only one end of these respective axes is visible in these figures.

Figure 2 shows that in the closed position the bore 22 does not coincide with the inlet port 15 and entry of urine is thereby prevented. Rotation of the operating lever 6 to the downward position indicated by dotted lines in Figure 2 moves the bore 22 into straight line alignment with the tubes 14 and 16 and thereby allows free flow of urine through the tube 14, port 15, bore 22, port 17 and tube 16. The lever 6 is however neatly disposed alongside the tubes 14 and 16 respectively when in both the closed and open positions and thus offers minimal risk of snagging on clothing or bedding. The angled end portion 10 of the tab 8 assists the user in getting a good finger grip on the operating lever 6 and the wide angle of rotation (about 155°) of the lever 6 between its open and closed positions assists in reducing the effort required to operate the valve.

## Claims

1. A fluid control valve which comprises a housing (2) having a generally cylindrical or frusto-conical inner surface with a fluid inlet port (15) and fluid outlet port (17) therein, a fluid inlet tube (14) and a fluid outlet tube (16) leading respectively to and from the fluid inlet port (15) and fluid outlet port (17), and a tap member (4) having a fluid flow conduit (22) therethrough and having an outer surface of complementary shape to the inner surface of the housing (2) so as to be movable therein about an axis of rotation between an open position in which its fluid flow conduit (22) is in alignment with the fluid inlet port (15) and fluid outlet port (17) and a closed position in which its fluid flow conduit (22) is out of alignment with at least one of the ports, the fluid flow conduit (22) being offset relative to the axis of rotation of the tap member (4), characterised in that the tap member (4) has a lever (6) attached thereto which is rotatable over a wide angle so as to be aligned close to the inlet tube (14) or outlet tube (16) in both the open and closed positions.

2. A fluid control valve as claimed in claim 1, in which the surface of rotation of the tap member (4) and the inner curved surface of the housing (2) form an interference fit.

3. A fluid control valve as claimed in claim 1 or claim 2, in which the surface of rotation of the tap member (4) and the inner curved surface of the housing (2) have a semi-fluid sealant and/or a lubricant between them.

4. A fluid control valve as claimed in any preceding claim, in which the housing (2) and tap member (4) have complementary circumferential ridges (26) and grooves at the open end (or ends) of the housing (2).

5. A fluid control valve as claimed in any preceding claim, in which the tap member (4) and lever (6) are formed from a single moulding.

6. A fluid control valve as claimed in any preceding claim, in which the housing (2), inlet and outlet ports (15, 17) and inlet and outlet tubes (14, 16) are formed from a single moulding.

7. A fluid control valve as claimed in any preceding claim, in which the fluid inlet and outlet tubes (14, 16) and the fluid flow conduit (22) through the tap member (4) are straight throughout their length.

8. A fluid control valve as claimed in any preceding claim, in which the fluid inlet and outlet tubes (14, 16) and the fluid flow conduit (22) are of substantially the same internal cross sectional area.

9. A fluid control valve as claimed in any preceding claim, in which the fluid inlet and outlet tubes (14, 16) and the fluid flow conduit (22) through the tap are aligned along a common axis.

10. A fluid control valve as claimed in claim 9, in which the common axis is at right angles to a plane passing through the axis of rotation of the tap member (4).

11. A fluid control valve as claimed in any preceding claim, in which the fluid flow conduit (22) is offset by a minimum of 20% of the radial distance from the axis of rotation to the circumference of the tap member (4).

12. A fluid control valve as claimed in any preceding claim, in which the cross sectional area of the fluid flow conduit (22) is 50 to 80% of the cross sectional area of the tap member (4).

## Patentansprüche

1. Fluidsteuerventil mit einem Gehäuse (2), das eine insgesamt zylindrische oder kegelstumpfförmige innere Oberfläche mit einem Fluideinlaß (15) und einem Fluidauslaß (17) aufweist, wobei ein Fluideinlaßrohr (14) und ein Fluidauslaßrohr (16) zu dem Fluideinlaß (15) führt bzw. von dem Fluidauslaß (17) ausgeht, und einem Ventilhahn (4), durch den ein Fluidkanal (22) hindurchgeht und der eine äußere Oberfläche aufweist, deren Form derjenigen der inneren Oberfläche des Gehäuses (2) entspricht, so daß er in dem Gehäuse um eine Drehachse drehbar ist zwischen einer offenen Position, in der der Fluidkanal (22) mit dem Fluideinlaß (15) und dem Fluidauslaß (17) ausgerichtet ist, und einer geschlossenen Position, in der der Fluidkanal (22) außerhalb der Ausrichtung mit wenigstens einem der Ein- und Auslässe liegt, welcher Fluidkanal (22) versetzt ist in bezug auf die Drehachse des Hahnes (4), dadurch **gekennzeichnet**, daß an dem Hahn (4) ein Hebel (6) angebracht ist, der über einen weiten Winkel derart drehbar ist, daß er dicht zu dem Einlaßrohr (14) oder Auslaßrohr (16) in der offenen und geschlossenen Stellung ausgerichtet ist.

2. Fluidsteuerventil nach Anspruch 1, bei dem die Rotations-Oberfläche des Hahnes (4) und die innere gekrümmte Oberfläche des Gehäuses (2) einen Preßsitz bilden.

3. Fluidsteuerventil nach Anspruch 1 oder 2, bei dem die Rotations-Oberfläche des Hahnes (4) und die innere gekrümmte Oberfläche des Gehäuses (2) ein halbfluidisches Dichtmittel und/oder ein Schmiermittel zwischeneinander aufweisen.

4. Fluidsteuerventil nach einem der vorhergehenden Ansprüche, bei dem das Gehäuse (2) und der Hahn (4) komplementäre umlaufende Rücken (26) und Nuten am offenen Ende (oder den Enden) des Gehäuses (2) aufweisen.

5. Fluidsteuerventil nach einem der vorhergehenden Ansprüche, bei dem der Hahn (4) und der Hebel (6) als zusammenhängendes Formteil ausgebildet sind.

6. Fluidsteuerventil nach einem der vorhergehenden Ansprüche, bei dem das Gehäuse (2), der Einlaß und Auslaß (15,17) und das Einlaß- und Auslaßrohr (14,16) als zusammenhängendes Formteil ausgebildet sind.

7. Fluidsteuerventil nach einem der vorhergehenden Ansprüche, bei dem das Fluideinlaß- und -auslaßrohr (14,16) und der Fluidkanal (22) durch den Hahn (4) über ihre gesamte Länge gerade sind.

8. Fluidsteuerventil nach einem der vorhergehenden Ansprüche, bei dem das Fluideinlaß- und -auslaßrohr (14,16) und der Fluidkanal (22) im wesentlichen den selben Innenquerschnitt aufweisen.

9. Fluidsteuerventil nach einem der vorhergehenden Ansprüche, bei dem das Fluideinlaß- und -auslaßrohr (14,16) und der Fluidkanal (22) durch den Hahn entlang einer gemeinsamen Achse ausgerichtet sind.

10. Fluidsteuerventil nach Anspruch 9, bei dem die gemeinsame Achse im rechten Winkel zu einer Ebene steht, die durch die Drehachse des Hahnes hindurchgeht.

11. Fluidsteuerventil nach einem der vorhergehenden Ansprüche, bei dem der Fluidkanal (22) wenigstens um 20% radial versetzt ist gegenüber der Drehachse in Richtung der Umfangsfläche des Hahnes (4).

12. Fluidsteuerventil nach einem der vorhergehenden Ansprüche, bei dem die Querschnittsfläche des Fluidkanals (22) 50-80% der Querschnittsfläche des Hahnes (4) beträgt.

## Revendications

1. Une vanne de réglage pour fluide, qui comporte un carter (2) présentant une surface interne cylindrique ou tronconique, en règle générale, avec un orifice d'entrée du fluide (15) et un orifice de sortie du fluide (17), un tube d'admission du fluide (14) et un tube de décharge du fluide (16) reliés respectivement à l'orifice d'entrée de fluide (15) et à l'orifice de sortie de fluide (17), et un élément de clé (4) présentant un conduit d'écoulement de fluide (22) et ayant une surface externe de forme complémentaire à la surface interne du carter (2), de façon à pouvoir s'y déplacer autour d'un axe de rotation entre une position ouverte, dans laquelle son conduit d'écoulement de fluide (22) est aligné avec l'orifice d'entrée de fluide (15) et l'orifice de sortie de fluide (17), et une position fermée dans laquelle son conduit d'écoulement de fluide (22) n'est plus aligné avec au moins l'un des orifices, le conduit d'écoulement de fluide (22) étant décalé par rapport à l'axe de rotation de l'élément de clé (4),
caractérisé en ce que l'élément de clé (4) possède un levier (6) Y fixé et qui est susceptible de pivoter angulairement dans une large mesure de façon à venir en alignement étroit avec le tube d'admission (14) ou le tube de décharge (16) à la fois dans les positions ouverte et fermée.

2. Une vanne de réglage pour fluide telle que revendiquée dans la revendication 1, dans laquelle la surface de rotation de l'élément de clé (4) et la surface interne incurvée du carter (2) forment un joint à ajustement serré.

3. Une vanne de réglage pour fluide telle que revendiquée dans la revendication 1 ou la revendication 2, dans laquelle la surface de rotation de l'élément de clé (4) et la surface interne incurvée du carter (2) ont entre elles un agent d'étanchéité semi-fluide et/ou un lubrifiant.

4. Une vanne de réglage pour fluide telle que revendiquée dans l'une des revendications précédentes, dans laquelle le carter (2) et l'élément de clé (4) ont des bords circonférentiels (26) et des rainures complémentaires à l'extrémité (ou aux extrémités) ouverte(s) du carter (2).

5. Une vanne de réglage pour fluide telle que revendiquée dans l'une des revendications précédentes, dans laquelle l'élément de clé (4) et le levier (6) viennent d'un moulage unique.

6. Une vanne de réglage pour fluide telle que revendiquée dans l'une quelconque des revendications précédentes, dans laquelle le carter (2), les orifices d'entrée et de sortie (15, 17) et les tubes d'admission et de décharge (14, 16) viennent d'un moulage unique.

7. Une vanne de réglage pour fluide telle que revendiquée dans l'une quelconque des revendications précédentes, dans laquelle les tubes d'admission et de décharge de fluide (14, 16) et le conduit d'écoulement de fluide (22) à travers l'élément de clé (4) sont rectilignes sur toute leur longueur.

8. Une vanne de réglage pour fluide telle que revendiquée dans l'une quelconque des revendications précédentes, dans laquelle les tubes d'admission et de décharge de fluide (14, 16) et le conduit d'écoulement de fluide (22) ont pratiquement la même surface interne de section transversale.

9. Une vanne de réglage pour fluide telle que revendiquée dans l'une quelconque des revendications précédentes, dans laquelle les tubes d'admission et de décharge de fluide (14, 16) et le conduit d'écoulement de fluide (22) à travers la clé sont alignés le long d'un axe commun.

10. Une vanne de réglage pour fluide telle que revendiquée dans la revendication 9, dans laquelle l'axe commun est orthogonal à un plan passant par l'axe de rotation de l'élément de clé (4).

11. Une vanne de réglage pour fluide telle que revendiquée dans l'une quelconque des revendications précédentes, dans laquelle le conduit d'écoulement de fluide (22) est décalé d'un minimum de 20 % de la distance radiale de l'axe de rotation à la circonférence de l'élément de clé (4).

12. Une vanne de réglage pour fluide telle que revendiquée dans l'une quelconque des revendications précédentes, dans laquelle la surface de la section transversale du conduit d'écoulement de fluide (22) est 50 à 80 % de la surface en section transversale de l'élément de clé (4).
